(19) Europäisches Patentamt
European Patent Office
Office européen des brevets

(11) Publication number: 0 549 156 A1

(12) # EUROPEAN PATENT APPLICATION

(21) Application number: 92310972.2

(22) Date of filing: 01.12.92

(51) Int. Cl.[5]: C07C 2/10, B01J 21/08, B01J 21/16, //C07C2/16, C07C2/26,C07C2/14,C07C2/18, B01J27/053,B01J31/04, B01J27/18,B01J27/25

(30) Priority: 23.12.91 US 816343
31.07.92 US 923204

(43) Date of publication of application:
30.06.93 Bulletin 93/26

(84) Designated Contracting States:
DE FR GB

(71) Applicant: TEXACO CHEMICAL COMPANY
3040 Post Oak Boulevard
Houston, Texas 77056(US)

(72) Inventor: Sanderson, John Ronald
15290 Faubion Trail
Leander, Texas 78641(US)
Inventor: Knifton, John Frederick
10900 Catskill Trail
Austin, Texas 78726(US)
Inventor: Marquis, Edward Thomas
9004 Collin Field Drive
Austin, Texas 78758(US)

(74) Representative: Brock, Peter William et al
UROUHART-DYKES & LORD 91 Wimpole Street
London W1M 8AH (GB)

(54) **Process for oligomerizing olefins using titanium salts or zirconium salts deposited on silicon dioxide.**

(57) Linear olefins having 10 to 24 carbon atoms can be converted into oligomers suitable for use as synthetic lubricant base stocks by contacting them at elevated temperature with a catalyst comprising titanium or zirconium oxide on a silica substrate, obtained by depositing a titanium or zirconium salt on the substrate, and then calcining at a temperature of at least 600 °C. In one embodiment the silica-supported titanium or zirconium oxide is sulphate-activated, and used as a blend with an acidic montmorillonite clay.

EP 0 549 156 A1

The invention relates to the preparation of synthetic lubricant base stocks, and more particularly to synthetic lubricant base stocks made by oligomerizing linear olefins.

Synthetic lubricants are prepared from man-made base stocks having uniform molecular structures and, therefore, well-defined properties that can be tailored to specific applications. Mineral oil base stocks, on the other hand, are prepared from crude oil and are complex mixtures of naturally occurring hydrocarbons. The greater uniformity of synthetic lubricants generally results in superior properties, for example, excellent thermal stability. As automobile engines are reduced in size to save weight and fuel, they run at higher temperatures, and therefore require a more thermally stable oil. Because lubricants made from synthetic base stocks have such properties as excellent oxidative/thermal stability, very low volatility, and good viscosity indices over a wide range of temperatures, they offer better lubrication and permit longer drain intervals, with less oil vaporization loss between oil changes.

Synthetic base stocks may be prepared by oligomerizing internal and alpha-olefin monomers to form a mixture of dimers, trimers, tetramers, and pentamers, with minimal amounts of higher oligomers. The unsaturated oligomer products are then hydrogenated to improve their oxidative stability. The resulting synthetic base stocks have uniform isoparaffinic hydrocarbon structures similar to those of high quality paraffinic mineral base stocks, but have the superior properties mentioned above, because of their greater uniformity.

Synthetic base stocks are produced in a broad range of viscosity grades. It is common practice to classify the base stocks by their viscosities, measured in centistokes (cSt) at 100 °C. Those base stocks with viscosities less than or equal to about 4 cSt are "low viscosity" base stocks, whereas base stocks having a viscosity of 40 to 100 cSt are "high viscosity" base stocks. Base stocks having a viscosity of 4 to 8 cSt are "medium viscosity" base stocks. The low viscosity base stocks generally are recommended for low temperature applications. Higher temperature applications, such as motor oils, automatic transmission fluids, turbine lubricants, and other industrial lubricants, generally require higher viscosities, such as those provided by medium viscosity base stocks (i.e. 4 to 8 cSt grades). High viscosity base stocks are used in gear oils and as blending stocks.

The viscosity of a base stock is determined by the length of the oligomer molecules formed during the oligomerization reaction. The degree of oligomerization is affected by the catalyst and reaction conditions employed during the oligomerization reaction. The length of the carbon chain of the monomer starting material also has a direct influence on the properties of the oligomer products. Fluids prepared from short-chain monomers tend to have low pour points and moderately low viscosity indices, whereas fluids prepared from long-chain monomers tend to have moderately low pour points and high viscosity indices. Oligomers prepared from long-chain monomers generally are more suitable than these prepared from shorter-chain monomers for use as medium viscosity synthetic lubricant base stocks.

One known approach to oligomerizing long-chain olefins to prepare synthetic lubricant base stocks is to contact the olefin with boron trifluoride, together with a promoter. See, for example, US-A-4400565, US-A-4420646, US-A-4420647 and US-A-4434308. However, boron trifluoride gas ($BF_3$) is a pulmonary irritant, and breathing the gas or fumes formed by hydration of the gas with atmospheric moisture, poses hazards which are preferably avoided. Additionally, the disposal/neutralization of $BF_3$ raises environmental concerns. Thus, a method for oligomerizing long-chain olefins using a less hazardous catalyst constitutes a substantial improvement in the art.

Our prior European Application 92305940.6 (EP-A-            ) discloses an oligomerization process using a catalyst obtained by depositing a halogen-free titanium or zirconium salt on clay (e.g. cation-exchanged montmorillonite).

Our prior European Application 92309842.0 (EP-A-            ) discloses an oligomerization process wherein the catalyst is a sulphate-activated Group IV oxide (e.g. titanium oxide or zirconium oxide).

We have now discovered, surprisingly, that a high conversion of olefin into oligomer may be obtained by contacting the olefin with a catalyst prepared by depositing a non-halogenated, non-polymeric, non-organometallic titanium salt on a substrate of silicon dioxide and calcining the substrate and deposited salt. catalysts so prepared are as active as, yet less expensive than, the corresponding unsupported, essentially pure titanium or zirconium oxide. Additionally, the process of this embodiment of the present invention results in a high percentage of dimers, i.e., a high dimer to trimer ratio. A high proportion of dimers is often desirable when preparing a synthetic lubricant base stock from olefins having 14 or more carbon atoms. In the absence of the high dimer to trimer ratio obtained using this embodiment of the present invention, a synthetic lubricant base stock prepared from olefins having 14 or more carbon atoms would contain a higher percentage of high molecular weight oligomers and may have too great a viscosity for some applications.

The present invention relates to a process for the oligomerization of linear olefins having 10 to 24 carbon atoms by contacting the olefins at elevated temperature with a catalyst comprising titanium oxide or zirconium oxide on a silicon dioxide substrate, obtained by depositing a non-halogenated, non-polymeric, non-organometallic titanium or zirconium salt on the substrate, and calcining at a temperature of at least 600 ° C.

According to one embodiment of the invention, the titanium oxide or zirconium oxide supported on silicon dioxide (a) is sulphate-activated and is physically blended with an acidic montmorillonite clay (b) having a moisture content up to 20 wt.%, a residual acidity of 3 to 30 mg KOH/g, and a surface area of at least 300 $m^2/g$, wherein the weight ratio of (a) to (b) is from 1:1000 to 1:4, preferably 1:50 to 1:9.

It is preferred that the olefins are oligomerized at a temperature of 120 to 250 ° C, more preferably from 140 to 180 ° C, and at a pressure of 0.1 to 7.0 MPa (atmospheric to 1000 psig).

The olefin monomer feedstocks used in the present invention may be alpha-olefins having the formula $R''CH=CH_2$, where R'' is an alkyl radical having 8 to 22 carbon atoms, or internal olefins having the formula RCH = CHR', where R and R' are the same or different alkyl radicals having 1 to 21 carbon atoms, provided that the total number of carbon atoms in any one olefin is from 10 to 24, inclusive. Alpha-olefins are preferred. A preferred range for the total number of carbon atoms in any one olefin molecule is 12 to 18, inclusive, with an especially preferred range being 14 to 16, inclusive. Mixtures of internal and alpha-olefins may be used, as well as mixtures of olefins having different numbers of carbon atoms, provided that the total number of carbon atoms in any one olefin is from 10 to 24, inclusive. The alpha and internal-olefins that may be oligomerized according to the present invention may be obtained by processes well-known to those skilled in the art and are commercially available.

The oligomerization reaction may be represented by the following general equation:

$$nC_mH_{2m} \xrightarrow{\text{catalyst}} C_{mn}H_{2mn}$$

where n represents moles of monomer and m represents the number of carbon atoms in the monomer. Thus, the oligomerization of 1-tetradecene may be represented as follows:

$$nC_{14}H_{28} \xrightarrow{\text{catalyst}} C_{14n}H_{28n}$$

The reaction occurs sequentially. Initially, olefin monomer reacts with olefin monomer to form dimers. Some of the resulting dimers then react with additional olefin monomer to form trimers, and so on. This results in an oligomer product distribution that varies with reaction time. As the reaction time increases, the olefin monomer conversion increases, and the selectivities for the heavier oligomers increase. An advantage of the present invention, particularly when oligomerizing olefins having 14 or more carbon atoms, is that a high percentage of trimer (relative to dimer) is observed. Generally, each resulting oligomer contains one double bond.

According to the present invention, oligomers of long-chain olefins may be prepared using catalysts easily and economically prepared by the following steps: (a) depositing a non-halogenated, non-polymeric, non-organometallic titanium or zirconium salt on a silicon dioxide substrate, and then (b) calcining. The silicon dioxide substrate may be any of the various - primarily amorphous - forms of $SiO_2$, (see Kirk-Othmer, Encyclopedia of Chemical Technology, 3rd. ed., vol. 20, pp. 748-764 (1981)). Silica gels, which contain three-dimensional networks of aggregated silica particles of colloidal dimensions, are preferred. Silica gels are commercially available in at least the following Tyler mesh sizes: 3-8 (2.38-6.73 mm); 6-16 (1.0-3.36 mm); 14-20 (0.84-1.19 mm); 14-42 (0.35-1.19 mm); and 28-200 (0.074-0.595 mm) and greater. A suitable commercially available silica gel is the Grade 12, 28-200 mesh (0.074-0.595 mm), silica gel available from Aldrich Chemical Co., Inc.

The silica gel should be added to a solution of 0.05 to 25 wt. %, preferably from 10 to 20 wt. %, of non-halogenated, non-polymeric, non-organometallic titanium or zirconium salt in water. The ratio of silica gel to titanium or zirconium salt solution should be sufficient to provide a catalyst having a quantity of titanium or zirconium salt deposited thereon of from 0.05 to 15 wt. %, preferably from 0.05 to 5.0 wt. %. The silica gel

should remain in the titanium salt or zirconium salt solution for a period of time, and under agitation to the extent necessary, to meet these requirements, and then filtered and dried. Optionally, after filtration, the silica gel may be washed with distilled water before being dried, preferably under mild conditions.

Preferably, the non-halogenated, non-polymeric, non-organometallic titanium or zirconium salt is a simple, two component salt. If a titanium salt is chosen, it is preferably titanium sulphate, titanium citrate, titanium nitrate, or titanium phosphate, most preferably titanium sulphate. Non-halogenated, non-polymeric, non-organometallic titanyl compounds are also acceptable titanium salts for purposes of this invention. Preferred zirconium salts include zirconium sulphate, zirconium citrate, zirconium nitrate, and zirconium acetate. Non-halogenated, non-polymeric, non-organometallic zirconyl compounds are also acceptable for purposes of this invention. Of the zirconium salts, zirconium sulphate is especially preferred. Zirconium salts are preferred over titanium salts.

The silicon dioxide substrate having the titanium or zirconium salt deposited thereon is calcined before use as an oligomerization catalyst. Calcination in air or in an inert gas environment, such as nitrogen, is conducted at a temperature of at least 600°C, but below the temperature at which thermal destruction leads to catalyst deactivation. Typically, the silicon dioxide substrate are calcined for 1 to 24 hours, preferably from 15 to 20 hours, at a temperature of at least 600°C, preferably from 600 to 800°C. Good results were obtained after calcination at a temperature of about 650°C for 18 hours. Temperatures above 900°C should be avoided.

According to one embodiment of the invention, the above described catalyst is employed as a physical blend with an acidic montmorillonite clay.

The Applicants have discovered, surprisingly, that excellent yields of oligomers are obtained using these catalysts. The high surface area of the silicon dioxide support unexpectedly permits one skilled in the art to obtain substantially the same benefits as are obtained by blending unsupported sulphate-activated titanium or zirconium oxides with acidic clays, but requires less of the expensive sulphate-activated oxide. The catalyst contains the silicon dioxide-supported titanium or zirconium oxide and the acidic montmorillonite clay in a weight ratio of silicon dioxide-supported titanium or zirconium oxide to clay of 1:1000 to 1:4. More preferably 1:50 to 1:9, and most preferably from 1:50 to 1:20.

The montmorillonite clays used in the present inventive process fall in the group of smectite clays. Smectite clays have unusual intercalation properties that afford them a high surface area. Smectites comprise layered sheets of octahedral sites between sheets of tetrahedral sites, where the distance between the layers can be adjusted by swelling, using an appropriate solvent. Three-layered sheet-type smectites include the montmorillonites. The montmorillonite structure may be represented by the following formula:

$$M^{n+}_{x/n} \cdot yH_2O(Al_{4-x}Mg_x)(Si_8)O_{20}(OH)_4$$

where M represents the interlamellar (balancing) cations, normally sodium or lithium; and x, y and n are integers.

Montmorillonite clays may be acid-activated by such mineral acids as sulphuric acid or hydrochloric acid. Mineral acids activate montmorillonites by attacking and solubilizing structural cations in the octahedral layers. This opens up the clay structure and increases surface area. These acid-treated clays act as strong Bronsted acids. Suitable acid-treated clays include, for example, acidic calcium montmorillonite clays. The clays have a moisture content up to 20 wt. %, a residual acidity of 3 to 30 mg KOH/g (when titrated to a phenolphthalein end point), and a surface area of at least 300 $m^2/g$. Illustrative examples of commercially available acid-treated clays include Engelhard Corporation's Grade F24, having a moisture content of 12 wt. %, a residual acidity of 16 mg KOH/g, and a surface area of 350 $m^2/g$; Grade F124, having a moisture content of 4 wt. %, a residual acidity of 14 mg KOH/g, and a surface area of 350 $m^2/g$; Grade F13, having a moisture content of 12 wt. %, a residual acidity of 15 mg KOH/g, and a surface area of 300 $m^2/g$; Grade F113, having a moisture content of 4 wt. %, a residual acidity of 15 mg KOH/g, and a surface area of 300 $m^2/g$; and Grade F224, having virtually no moisture, and having a residual acidity of 5 mg KOH/g, and a surface area of 350 $m^2/g$.

Preferably, the acidic montmorillonite clay is activated by heat treatment before being blended with the sulphate-activated silica-supported, titanium or zirconium oxide component. We have found, surprisingly, that heat treatment of the clay component before it is blended with the sulphate-activated oxide component

causes the catalyst to be more active and produce higher olefin conversions. Additionally, clays heat treated in this manner are more stable, remaining active during the oligomerization reaction for a longer period. The clays may be heat treated at temperatures of 50 to 400 ° C, with or without the use of a vacuum. A more preferred temperature range is 50 to 300 ° C. Optionally, an inert gas may be used during heat treatment as well. Preferably, the clay should be heat treated under conditions and for a length of time that will reduce the water content of the clay to approximately 1 wt. % or less.

The sulphate-activated oxide component of the blended catalyst may be prepared by treating the silica-supported titanium or zirconium oxide, prepared as described above, with a sulphate compound. Preferably, the sulphate compound is ammonium sulphate, ammonium hydrogen sulphate, sulphuric acid, sulphur trioxide, sulphur dioxide, or hydrogen sulphide. Especially preferred sulphating agents are ammonium sulphate and sulphuric acid. Said agents may be employed neat, or as an aqueous, ketonic, alcoholic, or ether solution, but preferably as an aqueous solution. the sulphating agents also may be employed as mixtures of the sulphating agents listed above. Excess sulphating agent may be removed by known procedures, such as filtration.

Alternatively, the sulphate-activated titanium or zirconium oxide catalyst is obtained as a result of using titanium or zirconium sulphate to deposit the titanium or zirconium on the silica substrate in the manner defined above.

In a more specific embodiment, the oxide component is treated with sulphuric acid by adding said acid neat or, if desired, diluted with distilled water, to the oxide extrudates. The slurry is then mixed for 1 to 24 hours, filtered, washed, and calcined in a stream of air for 1 to 24 hours. The prepared sulphuric acid-treated oxide should then have a titratable acidity of at least 0.1 meq/g.

The weight percentage of sulphuric acid to oxide is preferably such that the concentration of the sulphur in the formulated sulphate-activated Group IV oxide is in the range of 0.1 to 30 wt. %, although concentrations outside this range also may be employed.

The sulphate-activated oxide component is physically blended with the acidic montmorillonite clay in the proportions disclosed above. Optionally, the blended components may then be pelletized for easier handling. Diameters ranging from 0.794 mm (1/32 inch) to 9.525 mm (3/8 inch) possess desirable dimensions. The shape and dimensions of the pellets are not critical to the present invention; pellets of any suitable shape and dimensions may be used.

When cylindrical pellets of catalyst of the type described above are used in a fixed bed continuous flow reactor, the liquid hourly space velocity (LHSV) may be varied within wide limits (e.g., 0.1 to 10) in order to obtain a desired rate of conversion. Normally, space velocities of 0.5 to 2 will be employed.

The oligomerization reaction may be carried out either batchwise, in a stirred slurry reactor, or continuously, in a fixed bed continuous flow reactor. The catalyst concentration should be sufficient to provide the desired catalytic effect. The temperatures at which the oligomerization may be performed are from 50 to 300 ° C, preferably from 120 to 250 ° C, more preferably from 140 to 180 ° C. An especially preferred range is from 160 to 180 ° C. At temperatures of 200 ° C or above, the amount of unsaturation remaining in the products of the oligomerization reaction may decrease, thus reducing the degree of hydrogenation necessary to remove unsaturation from the base stocks. However, at temperatures above 200 ° C, the olefin conversion may decrease. The dimer to trimer ratio may increase. Applicants have found that the addition of a hydrocarbon containing a tertiary hydrogen, such as methylcyclohexane, may further reduce the amount of unsaturation present in the base stocks. One skilled in the art may choose the reaction conditions most suited to the results desired for a particular application. The reaction may be run at pressures of 0.1 to 7.0 MPa (0 to 1000 psig).

Following the oligomerization reaction, the unsaturated oligomers may be hydrogenated to improve their thermal stability and to guard against oxidative degradation during their use as lubricants. Hydrogenation processes known to those skilled in the art may be used to hydrogenate the oligomers. A number of metal catalysts are suitable for promoting the hydrogenation reaction, including nickel, platinum, palladium, copper, and Raney nickel. These metals may be supported on a variety of porous materials such as kieselguhr, alumina, or charcoal, or they may be formulated into a bulk metal catalyst. A particularly preferred catalyst for this hydrogenation is a nickel-copper-chromia catalyst described in US-A-3152998. Other known hydrogenation procedures are disclosed in US-A-4045508; US-A-4013736; US-A-3997621 and US-A-3997622.

Unreacted monomers may be removed either before or after the hydrogenation step. Optionally, unreacted monomers may be stripped from the oligomers before hydrogenation and recycled to the catalyst bed for oligomerization. The removal or recycle of unreacted monomers or, if after hydrogenation, the removal of non-oligomerized alkanes, should be conducted under mild conditions using vacuum distillation procedures known to those skilled in the art. Distillation at temperatures exceeding 250 ° C may

cause the oligomers to break down in some fashion and come off as volatiles. Preferably, therefore, the reboiler or pot temperature should be kept at or below 225°C when stripping out the monomers. Procedures known by those skilled in the art to be alternatives to vacuum distillation also may be employed to separate unreacted components from the oligomers.

While it is known to include a distillation step after the hydrogenation procedure to obtain products of various 100°C viscosities, it is preferred in the method of the present invention that no further distillation (beyond monomer flashing) be conducted. In other words, the monomer-stripped, hydrogenated bottoms are the desired synthetic lubricant components. Thus, the method of this invention does not require the costly, customary distillation step, yet, surprisingly, produces a synthetic lubricant component that has excellent properties and that performs in a superior fashion. However, in some contexts, one skilled in the art may find subsequent distillation useful in the practice of this invention.

The invention will be further illustrated by the following Examples.

## EXAMPLES

In the Examples 1 to 22 detailed below, the following procedures were used:

### Preparation of Catalysts

Cat No. 1: Silica gel (Grade-12, 0.074-0.595 mm (28 - 200 mesh), 200 g) was placed in a beaker and covered with 10 % zirconium sulphate solution. This was mixed well, and let stand for an hour. The beaker was then placed in an oven, which was heated to 400°C, and held at this temperature for 18 hours. The white solid was cooled under nitrogen and placed in a stoppered bottle.

Cat No. 2: Silica gel as used for Catalyst No. 1 (400 g) in a crucible was covered with 500 g 20 % zirconium sulphate solution. This was mixed well, and let stand for an hour. The crucible was then placed in an oven and heated to 650°C for 18 hours. The solid was cooled under nitrogen and placed in a stoppered bottle.

### Oligomerization of Olefins

Olefin and catalyst were charged to a flask equipped with a overhead stirrer, thermometer, heating mantle, and a water-cooled condenser ($N_2$ purge). The mixture was vigorously stirred and heated to the desired temperature for the desired time. The mixture was then cooled to ambient temperature and filtered with suction. The liquid was analyzed by liquid chromatography. The results obtained are detailed in Table 1 below.

It will be apparent from consideration of the results set out in Table 1 below that calcining the catalyst (Catalyst 2) at a temperature above 600°C processes an improved conversion when compared with a catalyst (Catalyst 1) obtained by calcining at a temperature below 600°C.

TABLE 1

| Ex No | Olefin(s) (by carbon number) | (g) of Olefin | Catalyst | Amount of Catalyst (g) | Time/Temp (Hr)/(°C) | Olefin Con. (%) | M (%) | D (%) | T+ (%) | D/T+ Ratio |
|---|---|---|---|---|---|---|---|---|---|---|
| 1* | C-14 α | 100 | $ZrO_2 \bullet SiO_2$ (Cat. #1) | 10 | 5/160 | 26.1 | 73.9 | 23.5 | 2.60 | 9.04 |
| 2* | C-10 α | 100 | $ZrO_2 \bullet SiO_2$ (Cat. #1) | 10 | 4/140 | 31.9 | 68.1 | 29.0 | 2.94 | 9.86 |
| 3* | C-14 α | 100 | $ZrO_2 \bullet SiO_2$ (Cat. #1) | 10 | 5/160 | 45.5 | 54.5 | 38.6 | 6.84 | 5.64 |
| 4* | C-10 α | 100 | $ZrO_2 \bullet SiO_2$ (Cat. #1) | 10 | 4/140 | 34.4 | 65.6 | 31.2 | 3.13 | 9.87 |
| 5 | C-14 α | 100 | $ZrO_2 \bullet SiO_2$ (Cat. #2) | 10 | 6/140 | 52.1 | 48.0 | 43.6 | 8.51 | 5.12 |
| 6 | C-14 α | 100 | $ZrO_2 \bullet SiO_2$ (Cat. #2) | 10 | 5/160 | 58.9 | 41.1 | 48.7 | 10.2 | 4.77 |
| 7 | C-14 α | 100 | $ZrO_2 \bullet SiO_2$ (Cat. #2) | 10 | 4/180 | 67.1 | 32.9 | 54.2 | 12.8 | 4.23 |
| 8 | C-14 α | 100 | $ZrO_2 \bullet SiO_2$ (Cat. #2) | 20 | 5/160 | 74.9 | 25.1 | 55.5 | 19.4 | 2.86 |
| 9 | C-14 α | 100 | $ZrO_2 \bullet SiO_2$ (Cat. #2) | 5 | 5/160 | 35.1 | 64.9 | 30.8 | 4.27 | 7.21 |
| 10 | C-13,14 I | 100 | $ZrO_2 \bullet SiO_2$ (Cat. #2) | 10 | 4/180 | 5.71 | 94.3 | 5.71 | – | – |
| 11 | C-15,18 I | 100 | $ZrO_2 \bullet SiO_2$ (Cat. #2) | 10 | 4/180 | 16.3 | 83.7 | 16.3 | – | – |
| 12 | C-14,16 α | 100 | $ZrO_2 \bullet SiO_2$ (Cat. #2) | 10 | 4/180 | 63.0 | 37.0 | 52.2 | 10.8 | 4.83 |
| 13 | C-10 α | 100 | $ZrO_2 \bullet SiO_2$ (Cat. #2) | 10 | 5/160 | 79.8 | 20.2 | 57.3 | 22.5 | 2.55 |
| 14 | C-12 α | 100 | $ZrO_2 \bullet SiO_2$ (Cat. #2) | 10 | 5/160 | 62.5 | 32.5 | 51.0 | 16.4 | 3.11 |
| 15 | C-14 α | 100 | $ZrO_2 \bullet SiO_2$ (Cat. #2) | 10 | 5/160 | 57.7 | 42.3 | 48.0 | 9.69 | 4.95 |
| 16 | C-14 α | 100 | $ZrO_2 \bullet SiO_2$ (Cat. #2) | 10 | 5/160 | 62.2 | 37.8 | 50.0 | 10.6 | 4.72 |
| 17 | C-10 α | 100 | $ZrO_2 \bullet SiO_2$ (Cat. #2) | 10 | 5/160 | 75.1 | 24.9 | 56.6 | 18.4 | 3.08 |
| 18 | C-10 α | 100 | $ZrO_2 \bullet SiO_2$ (Cat. #2) | 10 | 4/180 | 80.2 | 19.8 | 59.2 | 21.1 | 2.81 |
| 19 | C-10 α | 100 | $ZrO_2 \bullet SiO_2$ (Cat. #2) | 20 | 5/160 | 79.8 | 20.2 | 56.9 | 22.9 | 2.48 |
| 20 | C-10 α | 100 | $ZrO_2 \bullet SiO_2$ (Cat. #2) | 5 | 5/160 | 58.4 | 41.6 | 49.5 | 8.9 | 5.56 |
| 21 | C-10 α | 100 | $ZrO_2 \bullet SiO_2$ (Cat. #2) | 5 | 4/180 | 63.5 | 36.5 | 52.0 | 11.6 | 4.48 |
| 22* | C-10 α | 100 | $SiO_2$ | 10 | 4/180 | ~0 | ~100 | – | – | – |

I = Internal; Con. = Conversion; M = Monomer; D = Dimer; and Trimer+ = Trimer + Tetramer + Pentamer, etc.

* = Comparison Example

In the examples detailed in Table 2 below, the following procedures were used:

Aldrich silica gel (Grade 12, as used in the previous Examples) in a crucible, was treated with 500 g of 20% $ZrSO_4$ in demineralized water. The crucible was placed in an oven, heated to 650 °C, and held at this temperature for 18 hours under a flow of nitrogen. The white solid was cooled under nitrogen and stored in a stoppered bottle until used. Atomic absorption analysis showed 4.8 % zirconium.

Oligomerization of Olefins

Samples of the $ZrO_2/SiO_2$ catalyst components prepared above were ground to a fine powder with Engelhard Corporation's Grade F13 acidic montmorillonite clay, in the proportions listed in Table 2 below. Olefin and blended catalyst were charged to a flask equipped with an overhead stirrer, thermometer, heating mantle, and a water-cooled condenser ($N_2$ purge). The mixture was vigorously stirred and heated to the desired temperature for the desired time. the mixture was then cooled to ambient temperature and filtered with suction. The liquid was analyzed by liquid chromatography. The results obtained are detailed in Table 2 below.

TABLE 2

| Ex. No. | Olefin (by carbon number) | (g) of Olefin | Catalyst | (g) of Catalyst Components | Time/Temp. (Hr)/(°C) | Con. (%) | D/T+ Ratio |
|---|---|---|---|---|---|---|---|
| 23 | C-14 α | 100 | Dry Clay-13<br>$ZrO_2/SiO_2$ | 9.5<br>0.5 | 5/160 | 86.8 | 1.26 |
| 24 | C-14 α | 100 | Dry Clay-13<br>$ZrO_2/SiO_2$ | 8.0<br>2.0 | 5/160 | 86.2 | 1.46 |
| 25* | C-14 α | 100 | Dry Clay-13 | 10 | 5/160 | 83.1 | 1.81 |
| 26* | C-14 α | 100 | Dry Clay-13 | 10 | 5/160 | 84.6 | 1.73 |
| 27 | C-14 α | 100 | $ZrO_2/SiO_2$ | 10 | 5/160 | 54.3 | 4.95 |
| 28 | C-14 α | 100 | Dry Clay-13<br>$ZrO_2/SiO_2$ | 9.8<br>0.2 | 5/160 | 84.7 | 1.57 |
| 29 | C-14 α | 100 | Dry Clay-13<br>$ZrO_2/SiO_2$ | 9.5<br>0.5 | 5/160 | 85.9 | 1.44 |
| 30 | C-14 α | 100 | Dry Clay-13<br>$ZrO_2/SiO_2$ | 9.0<br>1.0 | 5/160 | 85.3 | 1.38 |
| 31 | C-14 α | 100 | Dry Clay-13<br>$ZrO_2/SiO_2$ | 8.5<br>1.5 | 5/160 | 84.0 | 1.53 |
| 32 | C-14 α | 100 | Dry Clay-13<br>$ZrO_2/SiO_2$ | 8.0<br>2.0 | 5/160 | 83.8 | 1.45 |
| 33 | C-14 α | 100 | Dry Clay-13<br>$ZrO_2/SiO_2$ | 9.5<br>0.5 | 4/180 | 89.1 | 1.16 |
| 34 | C-14 α | 100 | Dry Clay-13<br>$ZrO_2/SiO_2$ | 9.0<br>1.0 | 4/180 | 85.2 | 2.05 |
| 35* | C-1314 I | 100 | Dry Clay-13 | 10 | 5/160 | 46.3 | 4.02 |
| 36 | C-1314 I | 100 | Dry Clay-13<br>$ZrO_2/SiO_2$ | 9.5<br>0.5 | 5/160 | 42.0 | 6.04 |
| 37 | C-1314 I | 100 | Dry Clay-13<br>$ZrO_2/SiO_2$ | 9.0<br>1.0 | 5/160 | 45.8 | 4.16 |
| 38 | C-1314 I | 100 | Dry Clay-13<br>$ZrO_2/SiO_2$ | 8.0<br>2.0 | 5/160 | 34.2 | 3.89 |
| 39* | C-1314 I | 100 | Dry Clay-13 | 10 | 4/180 | 73.4 | 3.95 |
| 40 | C-1314 I | 100 | Dry Clay-13<br>$ZrO_2/SiO_2$ | 9.5<br>0.5 | 4/180 | 79.8 | 3.0 |
| 41 | C-1314 I | 100 | Dry Clay-13<br>$ZrO_2/SiO_2$ | 9.0<br>1.0 | 4/180 | 83.7 | 1.77 |
| 42 | C-1314 I | 100 | Dry Clay-13<br>$ZrO_2/SiO_2$ | 8.0<br>2.0 | 4/180 | 81.1 | 2.76 |
| 43 | C-14 α | 100 | Dry Clay-13<br>$ZrO_2/SiO_2$ | 9.8<br>0.2 | 5/160 | 83.3 | 1.47 |
| 44 | C-14 α | 100 | Dry Clay-13<br>$ZrO_2/SiO_2$ | 9.6<br>0.4 | 5/160 | 83.6 | 1.55 |
| 45 | C-14 α | 100 | Dry Clay-13<br>$ZrO_2/SiO_2$ | 9.2<br>0.8 | 5/160 | 85.3 | 1.30 |
| 46 | C-14 α | 100 | Dry Clay-13<br>$ZrO_2/SiO_2$ | 9.0<br>1.0 | 5/160 | 87.7 | 1.15 |

α = Alpha Olefin; I = Internal Olefin; Con. = Olefin Conversion;
D/T+ = Ratio of Dimer : Trimer + Tetramer + Pentamer, etc.

* = Comparison Example

## Claims

1. A process for the oligomerization of linear olefins having 10 to 24 carbon atoms by contacting the olefins with a catalyst at elevated temperature, characterised in that the catalyst comprises titanium oxide or zirconium oxide on a silicon dioxide substrate, obtained by depositing a non-halogenated, non-polymeric, non-organometallic titanium or zirconium salt on the substrate, and calcining at a temperature of at least 600 ° C.

2. A process according to Claim 1, characterised in that the silicon dioxide substrate is silica gel.

3. A process according to Claim 1 or 2, characterised in that the titanium compound is titanium sulphate, titanium citrate, titanium nitrate, or titanium phosphate.

4. A process according to Claim 1 or 2, characterised in that the zirconium compound is zirconium sulphate, zirconium citrate, zirconium nitrate, or zirconium acetate.

5. A process according to any one of Claims 1 to 4, characterised in that the titanium oxide or zirconium oxide supported on silicon dioxide (a) is sulphate-activated and is physically blended with an acidic montmorillonite clay (b) having a moisture content up to 20 wt.%, a residual acidity of 3 to 30 mg KOH/g, and a surface area of at least 300 $m^2/g$, wherein the weight ratio of (a) to (b) is from 1:1000 to 1:4.

6. A process according to Claim 5, characterised in that the weight ratio of (a) to (b) is from 1:50 to 1:9.

7. A process according to any one of Claims 1 to 6, characterised in that the olefins are oligomerized at a temperature of 120 to 250 ° C and at a pressure of 0.1 to 7.0 MPa (atmospheric to 1000 psig).

8. A process according to Claim 7, characterised in that the temperature is from 140 to 180 ° C.

European Patent Office

# EUROPEAN SEARCH REPORT

Application Number

EP 92 31 0972

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | US-A-2 249 583 (C. L. THOMAS)<br>* page 2, left column, line 66 - page 2, right column, line 24 *<br>* page 3, left column, paragraph 1 -paragraph 2 *<br>--- | 1 | C07C2/10<br>B01J21/08<br>B01J21/16<br>//C07C2/16<br>C07C2/26<br>C07C2/14<br>C07C2/18<br>B01J27/053<br>B01J31/04<br>B01J27/18<br>B01J27/25 |
| A | US-A-4 024 080 (J. D. HOLMES)<br>* claims 1-2 *<br>----- | 1 | |
| | | | **TECHNICAL FIELDS SEARCHED (Int. Cl.5 )** |
| | | | C07C<br>C10G<br>B01J |

The present search report has been drawn up for all claims

| Place of search | Date of completion of the search | Examiner |
|---|---|---|
| THE HAGUE | 21 APRIL 1993 | J. VAN GEYT |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)